# EUROPÄISCHE PATENTSCHRIFT

(11) **EP 0 627 007 B1**
(45) Veröffentlichungstag und Bekanntmachung des Hinweises auf die Patenterteilung: **12.05.1999**
(21) Anmeldenummer: 93903936.8
(22) Anmeldetag: 09.02.1993
(51) Int. Cl.: C12P 7/40, C12P 7/26

(54) **VERFAHREN ZUR ENZYMATISCHEN OXIDATION VON (D)-MILCHSÄURE ZU BRENZTRAUBENSÄURE**
PROCESS FOR THE ENZYMATIC OXIDATION OF (D)-LACTIC ACID TO PYRUVIC ACIDS
PROCEDE D'OXYDATION ENZYMATIQUE DE (D)-ACIDES LACTIQUES EN ACIDE PYRUVIQUE

(30) Priorität: 21.02.1992 DE 4205391
(43) Veröffentlichungstag der Anmeldung: 07.12.1994
(73) Patentinhaber: BASF Aktiengesellschaft, 67063 Ludwigshafen (DE)
(72) Erfinder: SIMON, Helmut, D-8050 Freising (DE); SCHINSCHEL, Carsten, D-8037 Olching (DE)
(86) Internationale Anmeldenummer: EP9300310
(87) Internationale Veröffentlichungsnummer: WO9317120

(56) Entgegenhaltungen:
- EP-A- 0 260 611
- WO-A-88/08029
- DE-A- 3 226 888
- ANGEWANDTE CHEMIE, Band 99, No. 2, 1987; VON HAIKE SKOPAN et al., Seiten 139-141/

## Beschreibung

Die Erfindung betrifft ein verbessertes Verfahren zur Herstellung von Brenztraubensäure aus (D)-Milchsäure unter Verwendung eines Biokatalysators sowie die Herstellung von Mischungen von (L)-Milchsäure und Brenztraubensäure aus (D,L)-Milchsäure.

Bekannt sind Reduktionen mit Hilfe von Mikroorganismen, denen die Reduktionsäquivalente über einen externen Elektronenüberträger, der elektrochemisch regeneriert wird, zur Verfügung gestellt werden. Dazu kann eine Vielzahl von Mikroorganismen und als Elektronenüberträger eine Reihe von Farbstoffen und anderen Substanzen eingesetzt werden (DE-A 32 26 888 und DE-A 33 32 562).

Ebenfalls bekannt ist ein Verfahren zur elektroenzymatischen Herstellung von Verbindungen definierter optischer Reinheit. Das in EP-B-0 355 102 beschriebene Verfahren umfaßt mehrere Teilschritte, in deren Verlauf durch Oxidations- und Reduktionsreaktionen schließlich aus Pyruvat L-Milchsäure hergestellt wird. Als Reduktionsäquivalentdonor wird im Verfahren das teure und empfindliche NADH + H⁺ verwendet.

Aus der EP-A 260 611 ist die Oxidation von (D)-2-Hydroxycarbonsäuren zu 2-Ketocarbonsäuren mit Hilfe von Redoxenzymen, die die Reduktionsäquivalente unter anaeroben Bedingungen auf ein Viologen als externen Redoxmediator übertragen, bekannt. In dieser Patentanmeldung werden die beiden Viologene CAV (1,1'-Dicarboxamidomethyl-4,4'-dipyridinium-Dikation) und CYV (l,l'-Dicyanomethyl-4,4'-dipyridinium-Dikation) als Redoxmediatoren genannt, die elektrochemisch an einer Anode, mit Luftsauerstoff, mit Eisen(III)-Verbindungen oder mit Wasserstoffperoxid regeneriert werden können. Bei der Umsetzung von (D,L)-Milchsäure bzw. (D,L)-2-Hydroxyglutarat können jedoch nur die nicht reagierenden (L)-Formen isoliert werden. Brenztraubensäure und 2-Ketoglutarat werden durch das Verfahren metabolisiert und sind nicht isolierbar [Skopan et al., Angew. 99, 139-141 (1987)].

Die beschriebenen Viologene CAV und CYV besitzen außerdem eine beträchtliche Toxizität; weiterhin sind sie in dem bei der Reaktion verwendeten pH-Bereich nicht ausreichend stabil.

Es bestand daher die Aufgabe, ein Verfahren zur Herstellung von Brenztraubensäure bereitzustellen, das die oben erwähnten Nachteile nicht besitzt.

Es wurde nun gefunden, daß das Verfahren zur Herstellung von Brenztraubensäure aus (D)-Milchsäure unter Verwendung eines Biokatalysators besonders gut verläuft, wenn man die bei der Oxidation von (D)-Milchsäure entstehenden Elektronen auf Anthrachinonsulfonsäure überträgt.

Weiterhin wurde gefunden, daß sich mit diesem Verfahren aus (D,L)-Milchsäure Mischungen aus (L)-Milchsäure und Brenztraubensäure herstellen lassen.

Mit dem erfindungsgemäßen Verfahren können generell (D)-2-Hydroxycarbonsäuren zu den entsprechenden 2-Ketocarbonsäuren oxidiert werden. So können beispielsweise die von Schummer et al. (Tetrahedron 47, 9019-9034, 1991) beschriebenen 2-Hydroxycarbonsäuren als Substrat verwendet werden. Besonders geeignete (D)-2-Hydroxycarbonsäuren sind (D)-Glycerat, (D)-Gluconat, (D)-Galactonat, (D)-Gulonat, (D)-Ribonat, (D)-Xylonat und (L)-Mannonat, (L)-Arabinoat und Lactobionsäure (4-β-D-Galactosido)-D-gluconat). Nicht umgesetzt wird z.B. (L)-Gluconat, da C-2 (L) -Konfiguration besitzt. Ganz besonders geeignet ist das Verfahren zur Herstellung von Brenztraubensäure oder deren Salze (Pyruvate) aus (D)-Milchsäure bzw. deren Salzen. Wird (D,L)-Milchsäure als Ausgangsverbindung in dem Verfahren eingesetzt, so erhält man eine Mischung aus Pyruvat und (L)-Milchsäure, die sich leicht chemisch trennen läßt.

Die in dem Verfahren eingesetzten Biokatalysatoren können Mikroorganismen oder daraus hergestellte Enzympräparationen sein. Bevorzugt werden Mikroorganismen als Biokatalysatoren verwendet, wobei solche der Gattung Proteus, beispielsweise Proteus vulgaris DSM 30118 oder Proteus mirabilis DSM 30115, der Gattung Propionibacterium, beispielsweise Propionibacterium acidi-propionici DSM 20272, der Gattung Clostridium, beispielsweise Clostridium homopropionicum sp. nov. DSM 5847 oder der Gattung Paracoccus, beispielsweise Paracoccus denitrificans DSM 413 besonders bevorzugt werden.

Geeignete Mikroorganismen lassen sich leicht identifizieren, indem beispielsweise in einem Handversuch ihre Fähigkeit zur Oxidation von (D)-Lactat zu Pyruvat überprüft wird.

Als Enzym wird die 2-Hydroxycarboxylat-Viologen-Oxidoreduktase aus Proteus vulgaris oder Proteus mirabilis bevorzugt verwendet.

Die Mikroorganismen können separat angezüchtet werden und gegebenenfalls auch nach Lagerung als Zellsuspension oder als Enzympräparation anaerob in die Reaktionslösung gegeben werden. Es ist auch möglich das Substrat am Anfang oder Ende der Mikroorganismuszüchtung direkt dem Medium zuzugeben, so daß es nach Abschluß der Wachstumsphase umgesetzt wird. Diese Vorgehensweise eignet sich besonders wenn ein chemischer Elektronenakzeptor wie Dimethylsulfoxid verwendet wird.

Die mit dem erfindungsgemäßen Verfahren hergestellte 2-Ketocarbonsäure kann beispielsweise im Fall des Pyruvats von den verwendeten Mikroorganismen weiter metabolisiert werden. Daher ist es zweckmäßig, den Katobolismus des Pyruvats oder von Onsäuren wie (D)-Gluconat in Mikroorganismen zu inhibieren. Dies kann beispielsweise auf chemischem Weg durch Metall-komplexierende Substanzen wie Ethylendiamintetraessigsäure (EDTA) oder Antibiotika wie Tetracyclin erfolgen. Bevorzugt wird EDTA verwendet, wobei in der Regel Konzentrationen von 0,01 bis 10 mM eingesetzt werden.

Die bei der Oxidation der (D)-2-Hydroxycarbonsäuren entstehenden Elektronen werden auf einen Mediator übertragen.

Als Mediatoren kommen folgende Substanzgruppen in Frage:
1. Chinonfarbstoffe wie Anthrachinone oder Naphthochinone, z.B. Anthrachinonsulfonsäuren, Hydroxynaphthochinone,
2. Viologenfarbstoffe, z.B. Benzylviologen, CAV,
3. Triphenylmethan-Farbstoffe, z.B. Benzaurin, Aurin,
4. Phthalocyanine, z.B. Fe-, Cu- oder Co-Phthalocyanine,
5. Methinfarbstoffe, z.B. Astraphloxin,
6. Pyrrolfarbstoffe oder Porphyrinderivate, z.B. Metall-Chelatkomplexe dieser Verbindungen,
7. Pteridine oder Pteridone,
8. Flavine, z.B. Acriflavin, Lumiflavin,
9. Imidazol-Derivate, z.B. Metronidazol,
10. Metallkomplexe der Metalle der 6., 7. und 8. Nebengruppe, z.B. Ferrocen-Monocarbonsäuren,
11. Thiolate mit den Metallen der 6., 7. und 8. Nebengruppe,
12. Thiole, z.B. Dihydroliponsäure, Dithiothreitol, Glutathion,
13. Indophenole, z.B. 2,6-Dichlorophenolindophenol,
14. Indigofarbstoffe, z.B. Indigotetrasulfonat,
15. Naphthiazine, z.B. Rosindulin 2G,
16. Phenazine, z.B. Phenazinethosulfat, Pyocyanin,
17. Phenothiazine, z.B. Thionin, Toluidin Blau O,
18. Phenoxazine, z.B. Resorufin,
19. Metall-Chelatkomplexe mit Metallen der 8. Nebengruppe, z.B. Ethylendiamintetraessigsäure (EDTA) mit Eisen(II/III),
20. Mediator-Systeme aus Kombinationen der Gruppen 1 bis 19, z.B. Thionin und Eisen(II/III)-EDTA

In der vorliegende Erfindung werden die Mediatoren aus der Substanzgruppe 1 verwendet, wobei die Anthrachinonsulfonsäuren, z.B. Anthrachinon-2-sulfonsäure oder Anthrachinon-2,6-disulfonsäure, besonders gut geeignet sind.

Die Mediatoren werden in der Regel in katalytischen Mengen eingesetzt, wenn die Elektronen vom Mediator noch auf einen Elektronenakezptor übertragen werden.

Werden die Mediatoren aber auch als Elektronenakzeptor verwendet, so setzt man sie zweckmäßigerweise in äquimolaren Mengen zum zu oxidierenden Substrat ein.

Der in dem Verfahren reduzierte Mediator kann reoxidiert und somit erneut in dem Verfahren eingesetzt werden.

Die Reaktion läßt sich wie folgt beschreiben:

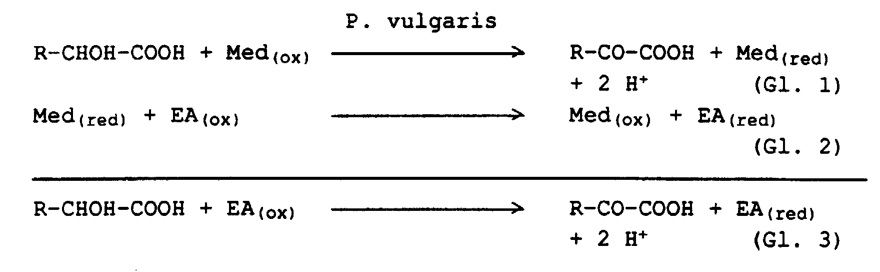

Die Reoxidation des Mediators (Med) bzw. Elektronenakzeptors (EA) (Gl. 2) kann auf verschiedene Weise erfolgen. Die jeweils gewählte Art der Reoxidation ist davon abhängig, welcher Mediator verwendet wird. Die Regeneration des Mediators kann kontinuierlich während der Umsetzung erfolgen. Eine andere Möglichkeit ist die Oxidation des äquimolar eingesetzten Mediators bzw. Elektronenakzeptors nach Beendingung der Umsetzung. Besonders vorteilhaft ist dies, wenn der reduzierte Elektronenakzeptor während der Umsetzung flüchtig ist, oder durch Destillation aus dem Reaktionsgemisch abgetrennt werden kann.

Zur Regeneration der Mediatoren eignen sich folgende Verfahren:
1. Der Mediator wird durch eine elektrochemische Zelle gepumpt und anodisch reoxidiert. Die Mikroorganismen können entweder mit durch die elektrochemische Durchflußzelle umgewälzt werden oder vorteilhafter durch eine Membran in Form eines Hollow-Fiber-Bündels oder als Immobilisat auf Sinterglas zurück gehalten werden, wobei sich die Aktivitätshalbwertszeit des Enzyms in den Mikroorganismen dadurch wesentlich erhöht.
2. Der Mediator wird durch Einblasen von Sauerstoff bzw. Luft in die Reaktionslösung regeneriert. Dazu kann die Reaktionslösung mit oder ohne Mikroorganismen durch ein Hollow-Fiber-Bündel gepumpt werden, das von außen mit Sauerstoff blasenfrei begast wird. Jedoch ist es auch hier vorteilhaft, die Mikroorganismen wie in Verfahren 1 zurückzuhalten. Wichtig bei dieser Methode ist, daß nur soviel Sauerstoff eingeblasen wird, wie gerade für die Regeneration der reduzierten Mediatormenge benötigt wird. Eine stete geringe Konzentration an reduziertem Mediator ist hierbei von Vorteil (anaerobe Bedingungen), da dann das Enzym nicht durch Sauerstoff geschädigt wird.
3. Der Mediator wird durch ein zweites Enzym in Proteus vulgaris oder Proteus mirabilis und einem weiteren Elektronenakzeptor regeneriert.
   Als Elektronenakzeptoren sind beispielsweise folgende Substanzgruppen geeignet:
   1. Sulfoxide, z.B. Dimethylsulfoxid (DMSO), Diethylsulfoxid, Tetramethylensulfoxid, DL-Methioninsulfoxid,
   2. N-Oxide, z.B. N-Methylmorpholin-N-oxid, Trimethylamin-N-oxid, Pyridin-N-oxid
   3. Fumarat,
   4. Nitrat,
   5. Chlorat,
   6. Thiosulfat, Tetrathiosulfat.
      Als besonders gut geeignete Elektroakzeptoren haben sich die N-Oxide, insbesondere N-Methylmorpholin-N-oxid erwiesen. Das bei der Reaktion entstehende N-Methylmorpholin läßt sich in wäßriger Lösung wieder zum N-Methylmorpholin-N-oxid oxidieren, indem man es beispielsweise mit Peroxiden wie Wasserstoffperoxid oder Percarbonsäure oder mit Sauerstoff umsetzt (Houben-Weyl, 1990, "Methoden der organischen Chemieé, Bd. 16a I, Seite 404-420).
      Der Elektronenakzeptor kann entweder in äquimolaren Mengen zum Substrat zugegeben werden oder in katalytischen Mengen und dann außerhalb des Reaktionsgemisches regeneriert werden; z.B. entweicht Dimethylsulfid (aus Dimethylsulfoxid) sehr leicht aus der wäßrigen Reaktionslösung und kann durch katalytische Oxidation mit Luftsauerstoff wieder in das Oxid (DMSO) überführt werden bzw. in die Reaktionslösung zurück gegeben werden.
4. Der Mediator wird durch einen zweiten Mikroorganismus, z.B. Paracoccus denitrificans DSM 413 regeneriert. Als Elektronenakzeptoren kommen hier Nitrat, Nitrit und Distickstoffoxid in Frage. Reduktionsprodukt ist hier molekularer Stickstoff, das als Gas aus der Reaktionslösung entweicht.

Die Reoxidations-Verfahren 1 bis 4 eignen sich für einen Batch-, Semibatch- und kontinuierlichen Betrieb.

Im allgemeinen eignen sich zur Durchführung der einzelnen Verfahren unter anaeroben Bedingungen Temperaturen von 10 bis 50, bevorzugt 25 bis 40°C. Der pH-Wert wird zweckmäßigerweise während der Reaktion gemessen und gegebenenfalls durch Zugabe von Natronlauge oder Salzsaure in einem Bereich von 8,5 bis 9,5 konstant gehalten. Die D-Milchsäurekonzentrationen bzw. D-Lactatkonzentrationen (pH 8 bis 9) liegen in der Regel bei 0,01 bis 0,7 M (1 bis 63 g/l). Die erforderliche Menge an D-Milchsäure kann am Anfang auf einmal oder während der Umsetzung in mehreren Portionen zugegeben werden. Die Gegenwart von L-Milchsäure stört dabei nicht; L-Milchsaure wird nicht zu Brenztraubensäure umgesetzt und bleibt daher unverändert. D-Milchsäure kann auch als Salz in die Reaktionslösung eingebracht werden. Verwendet werden können u.a. die Lithium-, Natrium-, Kalium- und Ammoniumsalze.

Die entstehende Brenztraubensäure bzw. das Pyruvat kann nach bekannten Verfahren (enzymatische Nachweismethode mit L-LDH/ NADH) quantifiziert werden [R. Czok, W. Lamprecht in H.U. Bergmeyer: Methoden der enzymatischen Analyse. 3. Aufl., Bd. I/II, 1407 bis 1411, Verlag Chemie, Weinheim (1974)]. Die Proben werden dazu mit Perchlorsäure (7 %) 1:1 versetzt, zentrifugiert, 1 h bei 95°C erhitzt und nach dem Abkühlen entsprechend verdünnt und analysiert.

Bei den Umsetzungen empfiehlt es sich den Ansatz abzubrechen, sobald die Brenztraubensäurekonzentration ihr Maximum erreicht. Die entstandene Brenztraubensäure kann dann nach bekannten Verfahren abgetrennt und gereinigt werden (siehe DE-A 37 33 157).

Das erfindungsgemäße Verfahren ermöglicht die Herstellung von Brenztraubensäure aus (D)-Milchsäure in hoher Ausbeute, ohne daß die sonst oft bei biokatalytischen Verfahren störende Metabolisierung des Endproduktes auftritt.

Die Erfindung wird durch folgende Beispiele veranschaulicht, wobei die als "illustrativ" bezeichneten Beispiele nicht unter das Anspruchsbegehren der Patentansprüche fallen:

### Beispiel 1

Anzucht von Proteus vulgaris mit höherer Bakterienfeuchtmasse pro Liter Medium ohne Aktivitätsverlust für das Enzym.

| Glucose-Medium (Medium A): | |
|---|---|
| Glucose | 10,0 g/l |
| Dikaliumhydrogenphosphat | 5,1 g/l |
| Hefe | 0,5-2,5 |
| Trypton | 5,0 g/l |
| Natriumformiat | 1,0 g/l* |
| Ammoniumchlorid | 0,17 g/l |
| Magnesiumsulfat (x 7 H₂O) | 0,05 g/l |
| Mangansulfat (x H2O) | 0,4 mg/l* |
| Eisensulfat (x 7 H2O) | 0,4 mg/l* |
| Dinatriummolybdat | 13,7 mg/l |
| Dinatriumselenit | 0,263 mg/l* |
| Calciumchlorid (x 2 H20) | 40,0 mg/l* |
| p-Aminobenzoesäure | 0,4 mg/l* |
| Biotin | 20,0 µg/l* |
| Wasser | ad 1 l |

Die Glucose und das Dikaliumhydrogenphosphat wurden getrennt vom restlichen Medium sterilisiert (20 min bei 121°C, anschließend wurde mit Stickstoff begast) und nach dem Abkühlen hinzugefügt. Der pH-Wert des Mediums betrug 7,5 bis 8,0.

Werden die Mikroorganismen nur für die Oxidation verwendet, können die mit einem Stern gekennzeichneten Mediumbestandteile weggelassen werden. Es zeigte sich dabei kein Aktivitätsverlust des Enzyms (2-Hydroxycarboxylat-Viologen-Oxidoreduktase) oder eine geringere Zellausbeute pro Liter. Das Medium wurde 0,5 bis 1,0 prozentig mit einer Proteus vulgaris-Vorkultur angeimpft. Bei 37°C mit pH-Regulierung bei 7,2 mit 2 bis 6 N Natronlauge (benötigt wurden ca. 6 g Natriumhydroxid/l Medium) war die Kultur in 15 bis 18 h ausgewachsen (Beginn der stationären Wachstumsphase). Die Bakterienfeuchtmasse betrug 6 bis 7 g/l bzw. 1,2 bis 1,4 g/l Trockenmasse.

Die Enzymaktivität der 2-Hydroxy-Carboxylat-Viologen-Oxidoreduktase betrug ca. 2,0 Units Lactat-Dehydrogenase/mg Protein (ca. 1 000 Units/g Bakterientrockenmasse); die der Dimethylsulfoxid-Reduktase betrug ca. 0,1 Units DMSO-Reduktase/mg Protein (ca. 50 Units/g Bakterientrockenmasse).

### Anzucht von Proteus vulgaris und Proteus mirabilis mit höheren Enzymaktivitäten

### (D,L)-Lactat-Medium (Medium B):

Wie Medium A ohne Glucose, jedoch mit 7,0 g/l (D,L)-Natriumlactat oder 3,5 g/l L-Natriumlactat (D-Lactat wird nicht verstoffwechselt) und 0,05 bis 0,1 M (3,9 bis 7,8 g/l) Dimethylsulfoxid (wurde nach dem Abkühlen des Mediums hinzugefügt). Der pH-Wert des Mediums betrug 7,5 bis 8,5. Das Medium wurde 0,5 bis 1,0 prozentig mit einer Proteus vulgaris-Vorkultur angeimpft. Bei 37°C war die Kultur in 18 bis 20 h ausgewachsen. Die Bakterienfeuchtmasse betrug ca. 2,5 g/l bzw. 0,5 g/l Trockenmasse. Für Proteus mirabilis wurden die doppelten Werte erreicht. Ähnliche Aktivitäten werden mit 50 mM Pyruvat oder Fumarat als Elektronenakzeptoren erzielt.

Die Enzymaktivität der 2-Hydroxy-Carboxylat-Viologen-Oxidoreduktase betrug ca. 4,0 Units Lactat-Dehydrogenase/mg Protein (ca. 2 000 Units/g Bakterientrockenmasse); die der Dimethylsulfoxid-Reduktase betrug ca. 0,4 Units DMSO-Reduktase/mg Protein (ca. 200 Units/g Bakterientrockenmasse). Die entsprechenden Zahlen für Proteus mirabilis betrugen ebenfalls 4,0 Units Lactat-Dehydrogenase/mg Protein. Der Wert für Dimethylsulfoxid-Reduktase erreichte 1,9 U/mg Protein (ca. 950 Units/g Bakterien-Trockenmasse).

### Beispiel 2

### Anzucht von Paracoccus denitrificans

| Glucose-Medium (Medium C): | |
|---|---|
| Glucose | 9,0 g/l |
| Kaliumnitrat | 10,0 g/l |
| Dinatriumhydrogenphosphat | 2,7 g/l |
| Kaliumdihydrogenphosphat | 4,1 g/l |
| Hefe | 0,1 g/l |
| Ammoniumchlorid | 1,6 g/l |
| Magnesiumsulfat (x 7 H₂O) | 0,25 g/l |
| Dinatriummolybdat (x 2H₂O) | 0,15 g/l |
| Mangansulfat (x H₂O) | 0,1 mg/l |
| Calciumchlorid (x 2 H₂O) | 20,0 mg/l |
| Eisenammoniumcitrat | 20,0 µM |
| Wasser | ad 1 l |

Das Medium C wurde wie Medium A sterilisiert. Das Eisenammoniumcitrat wurde nach dem Abkühlen hinzugefügt. Der pH-Wert des Mediums betrug ca. 7,3. Das Medium wurde 1,0 prozentig mit einer Paracoccus denitrificans-Vorkultur angeimpft. Bei 35°C ohne pH-Regulierung war die Kultur in 20 bis 24 h ausgewachsen (Beginn der stationären Wachstumsphase). Die Bakterienfeuchtmasse betrug ca. 8 g/l bzw. 1,6 g/l Trockenmasse.

### Beispiel 3

### Bildung von Pyruvat in der elektrochemischen Zelle mit Proteus vulgaris mit und ohne EDTA

In 70 ml Anolyt (entionisiertem Wasser) einer elektrochemischen Durchflußzelle mit einem Graphitfilz als Anode (und Kathode) wurden 35,5 mMol D-Lactat, 0,21 mMol Anthrachinon-2,6-disulfonsäure, 0,35 mMol EDTA und 362 mg Proteus vulgaris-Zellen (Trockenmasse) zugegeben. Über eine peristaltische Schlauchpumpe wurde die Reaktionslösung mit einer Flußrate von 18 l/h durch die elektrochemische Durchflußzelle umgewälzt. Als Volumenausgleich diente ein 90 ml Rührgefäß. Das Potential der elektrochemischen Durchflußzelle wurde bei -300 mV vs. SCE konstant gehalten, wobei ein maximaler Stromfluß von 0,5 A erreicht wurde. Die Temperatur betrug 35±0,5°C. Der pH-Wert wurde mit 4 N Natronlauge bei 8,5 konstant gehalten.

Nach 21,5 h waren 94,6 % (33,6 mMol) des D-Lactats zu Pyruvat oxidiert. 5,0 % (1,8 mMol) D-Lactat waren noch vorhanden. Der Umsatz berechnet nach Verbrauch der Natronlauge betrug ≥ 100 %; nach dem Stromverbrauch 94,9 %.

Der analoge Versuchsansatz ohne EDTA-Zugabe ergibt nach 21,5 h, daß 75,2 % (26,7 mMol) des D-Lactats zu Pyruvat oxidiert wurden. 13,0 % (4,6 mMol) D-Lactat waren noch vorhanden. Der Umsatz berechnet nach Verbrauch der Natronlauge betrug 108 %; nach dem Stromverbrauch ≥ 100 %.

### Beispiel 4

### Bildung von Pyruvat in der elektrochemischen Zelle mit Proteus vulgaris in einem Hollow-Fiber-Reaktor

In einem Rührgefäß wurden 129 mMol D-Lactat, 2,65 mMol Anthrachinon-2,6-disulfat, 0,27 mMol EDTA in 265 ml entionisiertem Wasser gelöst und 165 mg Proteus vulgaris-Zellen (Trockenmasse) zugegeben. Über eine Membran in Form eines Hollow-Fiber-Bündels wurde kontinuierlich ein Teil des Reaktionsvolumens abgetrennt, zur Reoxidation der Anthrachinon-2,6-disulfonsäure durch eine elektrochemische Zelle gepumpt und wieder in den Reaktionsraum zurückgeführt. Der pH-Wert wurde mit Natronlauge konstant gehalten. Die Temperatur betrug 35±0,5°C. Das Potential der elektrochemischen Durchflußzelle betrug -300 mV vs. SCE. Nach 143 h waren 95 % des D-Lactats zu Pyruvat oxidiert. 1,7 mMol (1,3 %) D-Lactat waren noch vorhanden.

### Beispiel 5

### Bildung von Pyruvat mit Proteus vulgaris und Regeneration des Mediators mit Sauerstoff

In einem Rührgefäß wurden 50,4 mMol D-Lactat, 1,0 mMol Anthrachinon-2,6-disulfonat, 0,32 mMol EDTA in 100 ml entionisiertem Wasser gelöst und 362 mg Proteus vulgaris-Zellen (Trockenmasse) zugegeben. Ein Teil des Reaktionsvolumens wurde kontinuierlich mit einer Flußrate von 15 l/h durch ein Hollow-Fiber-Bündel umgewälzt, der von außen mit einem Sauerstoffüberdruck von 0,5 bis 0,8 bar begast wurde. Die Reaktionslösung wurde blasenfrei begast. Die Temperatur betrug 35±0,5°C. Der pH-Wert wurde mit 4 N Natronlauge konstant gehalten. Nach 29 h waren 45 % (22,7 mMol) des D-Lactats zu Pyruvat oxidiert. Neben 33 % (16,4 mMol) D-Lactat konnten auch 5,9 mMol (11,7 %) Acetat nachgewiesen werden.

### Beispiel 6 (illustrativ)

### Bildung von Pyruvat mit Proteus vulgaris und Paracoccus denitrificans und Nitrat als Elektronenakzeptor

In einem Rührgefäß wurden 12,5 mMol D-Lactat, 0,05 mMol CAV, 0,5 mMol EDTA, 12,5 mMol Nitrat in 50 ml 0,3 M Tris/HCl-Puffer pH 8,5 gelöst und 90 mg Proteus vulgaris-Zellen (Trockenmasse) und 300 mg Paracoccus denitrificans-Zellen (Trockenmasse) zugegeben. Die Temperatur betrug 35±0,5°C. Der pH-Wert wurde mit 1 N Salzsäure bei 8,5 konstant gehalten. Nach 8,7 h waren 83 % (10,4 mMol) und nach 20,7 h 100 % (12,5 mmol) des D-Lactats zu Pyruvat oxidiert.

### Beispiel 7

### Pyruvat-Bildung aus D-Lactat mit N-Methylmorpholin-N-oxid als Elektronenakzeptor und Proteus vulgaris

Proteus vulgaris wurde in Medium B, wie in Beispiel 1 beschrieben, gezüchtet.

In einem Rührgefäß wurden 36,4 mMol D-Lactat, 0,35 mMol Anthrachinon-2,6-disulfonat, 0,70 mMol EDTA, 37,0 mMol N-Methylmorpholin-N-oxid in 70 ml entionisiertem Wasser gelöst und 181 mg Proteus vulgaris-Zellen (Trockenmasse) zugegeben. Die Temperatur betrug 40±0,5°C. Der pH-Wert wurde mit 2 N Salzsäure bei 8,5±0,1 konstant gehalten. Nach 4,0 h waren mehr als 99,5 % (36,3 mMol) des D-Lactats zu Pyruvat oxidiert.

### Beispiel 8

### Bildung von Pyruvat mit Proteus vulgaris und Dimethylsulfoxid als Elektronenakezptor

In einem Rührgefäß wurden 35,5 mMol D-Lactat, 0,21 mMol Anthrachinon-2,6-disulfonsäure, 0,21 mMol EDTA in 70 ml entionisiertem Wasser gelöst und 362 mg Proteus vulgaris-Zellen (Trockenmasse) zugegeben. 36,0 mMol Dimethylsulfoxid wurde während der Umsetzung in mehreren Portionen zugegeben. Die Temperatur betrug 38±0,5°C. Der pH-Wert wurde mit 2 N Natronlauge bei 8,5 konstant gehalten. Nach 8,5 h waren 95,5 % (33,9 mMol) des D-Lactats zu Pyruvat oxidiert. Mit Proteus mirabilis ließen sich ca. 1,5-fach raschere Umsetzungen erzielen.

Nach weiterer Zugabe von 35,5 mMol (Σ 71,0 mMol) D-Lactat und 36,0 mMol Dimethylsulfoxid konnte nach 29 h 50,9 mMol (71,7 %, 0,64 M) Pyruvat und 5 mMol (7,0 %) D-Lactat nachgewiesen werden. Vermutlich waren ca. 20 % des Pyruvats polymerisiert [Copper et al., Chem. Rev. 83, 321 bis 358 (1983)].

### Beispiel 9 (illustrativ)

### Bildung von Pyruvat mit Proteus vulgaris aus D-Lactat in Gegenwart von L-Lactat und Dimethylsulfoxid als Elektronenakzeptor.

In einem Rührgefäß wurden 35,5 mMol D-Lactat, 35,5 mMol L-Lactat, 0,21 mMol Anthrachinon-2,6-disulfonsäure und 0,21 mMol EDTA in 70 ml entionisiertem Wasser gelöst und 550 mg Proteus vulgaris-Zellen (Trockenmasse) zugegeben. 36,0 mMol Dimethylsulfoxid wurde während der Umsetzung in mehreren Portionen zugegeben. Die Temperatur betrug 38±0,5°C. Der pH-Wert wurde mit 2 N Natronlauge bei 8,5 konstant gehalten. Nach 3,6 h waren 100 % (35,5 mMol) des D-Lactats zu Pyruvat oxidiert. Das L-Lactat dagegen war noch vollständig vorhanden.

### Beispiel 10 (illustrativ)

### Bildung von Pyruvat mit mehrmals eingesetztem Biokatalysator (Proteus vulgaris) und Dimethylsulfoxid als Elektronenakzeptor

In einem Rührgefäß wurden 720 mg Proteus vulgaris-Zellen (Trockenmasse) mit 50 ml einer Reaktionslösung versetzt, in der 26,0 mMol D-Lactat, 0,15 mMol Anthrachinon-2,6-disulfonsäure und 0,25 mMol EDTA enthalten waren. 26,0 Dimethylsulfoxid wurde während der Umsetzung in mehreren Portionen zugegeben. Die Temperatur betrug 38±0,5°C. Der pH-Wert wurde mit 2 N Natronlauge bei 8,5 konstant gehalten. Bei einer D-Lactat-Konzentration von ≤ 2,0 % wurde die Umsetzung beendet. Die Proteus vulgaris-Zellen wurden durch Zentrifugation von der Reaktionslösung abgetrennt und wieder mit 50 ml Reaktionslösung versetzt. Der Biokatalysator wurde 6 mal hintereinander eingesetzt. Das Ergebnis zeigt die folgende Übersicht:

| Einsatz | Trockenmasse [mg] | Zeit [h] | Pyruvat [mMol] | D-Lactat [mMol] |
|---|---|---|---|---|
| 1. | 720 | 1,9 | 25,8 | 0,20 |
| 2. | 670 | 4,0 | 26,3 | 0,06 |
| 3. | 600 | 6,3 | 25,9 | 0,34 |
| 4. | 540 | 8,5 | 27,0 | 0,33 |
| 5. | 500 | 11,3 | 25,5 | 0,43 |
| 6. | 480 | 21,5 | 25,3 | 0,40 |

Die Mengenangaben an Pyruvat und D-Lactat beziehen sich auf die abzentrifugierte Reaktionslösung. Bei jedem Ansatz ging ein Teil der Proteus vulgaris-Zellen durch Proben und durch Abtrennen von der Reaktionslösung verloren. Ein Teil der Reaktionslösung bleibt bei jedem Ansatz in der Bakterienfeuchtmasse zurück, dies erklärt die etwas größere Menge an Pyruvat bei einigen Ansätzen.

### Beispiel 11 (illustrativ)

### Bildung von Pyruvat mit Proteus vulgaris im Anzuchtmedium

2 1 Medium A ohne Glucose, 100 mMol D-Lactat (kein L-Lactat) und 200 mMol Dimethylsulfoxid wurden 1,0 prozentig mit einer Proteus vulgaris-Vorkultur angeimpft. Die Wachstums- bzw. Umsetzungstemperatur lag bei 37±1°C; der pH-Wert bei 7,3 bis 7,8. Nach 21,5 h wurden 60,6 mMol (60,6 %) Pyruvat, 24,8 mMol (24,8 %) D-Lactat und 16,8 mMol (16,8 %) Acetat nachgewiesen. Dimethylsulfoxid war nicht mehr vorhanden.

### Beispiel 12

### Bildung von Pyruvat mit Proteus vulgaris und stöchiometrisch eingesetztem Mediator als Elektronenakzeptor

In einem Rührgefäß wurden 17,0 mMol D-Lactat, 17,0 mMol Anthrachinon-2,6-disulfonsäure und 0,35 mMol EDTA in 70 ml entionisiertem Wasser gelöst und 360 mg Proteus vulgaris) Zellen (Trockenmasse) zugegeben. Die Temperatur betrug 40±0,5°C. Der pH-Wert wurde mit 4 N Natronlauge bei 8,5 konstant gehalten. Nach 2,0 h waren 99,0 % (16,8 mMol) des D-Lactats zu Pyruvat oxidiert. 70 % (12 mMol) der Anthrachinon-2,6-disulfonsäure konnten durch Zentrifugation der Reaktionslösung bei ≤ 4°C zurück gewonnen werden und erneut verwendet werden. Die restlichen 30 % (5 mMol) blieben in der Reaktionslösung gelöst.

### Beispiel 13

### Bildung von Pyruvat mit immobilisierten Proteus vulgaris-Zellen auf Sinterglas-Raschigringen

In einem Festbettumlaufreaktor wurden 98,9 mMol D-Lactat, 1,25 mMol Anthrachinon-2,6-disulfat, 0,25 mMol EDTA in 250 ml entionisiertem Wasser gelöst und 28,2 mg Proteus vulgaris-Zellen (Trockenmasse, immobilisiert auf Sinterglas-Raschigringen) eingesetzt. Das Reaktionsvolumen wurde mit einer Flußrate von 20 l/h durch eine elektrochemische Zelle gewälzt. Der pH-Wert wurde mit 2 N Natronlauge konstant gehalten. Die Temperatur betrug 35±0,5°C. Das Potential der elektrochemischen Durchflußzelle betrug -300 mV vs. SCE. Nach 116,5 h waren 64,4 % (63,7 mMol) des D-Lactats zu Pyruvat oxidiert.

### Beispiel 14 (illustrativ)

### Umsetzung einer racemischen 2-Hydroxycarbonsäure mit Proteus vulgaris

In 40 ml Anolyt befanden sich 5 mg EDTA (zur Verhinderung der Decarboxylierung der entstehenden 2-oxo-4-phenyl-3-butensäure durch zweiwertige Metallionen), 17,7 mMol D,L-2-Hydroxy-4-phenyl-3E-butenoat und 0,64 mMol Anthrachinon-2,6-disulfonsäure. Die Temperatur betrug 37±0,5°C, die Pumpgeschwindigkeit durch die elektrochemische Durchflußzelle wurde auf 18 l/h eingestellt. Bei einem Potential von -527 mV vs. SCE in der elektrochemischen Durchflußzelle wurde ein Teil der oxidierten Anthrachinon-2,6-disulfonsäure anreduziert (Kathode und Anode wurden dazu umgepolt: der gelöste Sauerstoff im Anolyt wurde dadurch entfernt und somit eine anaerobe Reaktionslösung hergestellt). Danach wurde ein Potential von -327 mV vs. SCE eingestellt und 450 mg Proteus vulgaris-Zellen (Trockenmasse) in Form einer Suspension zugegeben, wobei ein maximaler Stromfluß von 0,1 A erreicht wurde. Nach 56 h zeigte ein Nullstrom an, daß alles Substrat umgesetzt wurde. Bis zum Ende der Reaktion wurden für die Titration des Anolyten (pH 8,5) 8,5 ml 2 N Natronlauge benötigt.

Umsatz nach:
- HPLC:: 8,2 mMol (92,6 %) 2-Oxo-4-phenyl-3-butensäure
7,7 mMol (87,0 %) L-2-Hydroxy-4-phenyl-3E-butenoat

- Berechnet nach Stromverbrauch:: 8,5 mMol (96,2 %)
- Berechnet nach NaOH-Verbrauch:: 8,5 mMol (96,2 %)

Die Trennung der 2-Ketocarbonsäure von der L-2-Hydroxycarbonsäure erfolgte durch Kristallisation in Diethylether und Chloroform und durch Trennung mittels MLCC (Multi-Layer-Coil-Chromatography).

### Beispiel 15

### Bildung von Pyruvat aus D-Lactat mit Propionibacterium acidi-propionici in einer elektrochemischen Zelle (Beispiel für einen anderen Mikroorganismus als Proteus vulgaris)

In 70 ml Anolyt (entionisiertem Wasser) einer elektrochemischen Durchflußzelle mit einem Graphitfilz als Anode (und Kathode) wurden 17,5 mMol D-Lactat, 0,21 mMol Anthrachinon-2,6-disulfonsäure, 0,35 mMol EDTA und 1 g Propionibacterium acidi-propionici-Zellen (Trockenmasse) zugegeben. Über eine peristaltische Schlauchpumpe wurde die Reaktionslösung mit einer Flußrate von 18 l/h durch die elektrochemische Durchflußzelle umgewälzt. Als Volumenausgleich diente ein 90 ml Rührgefäß. Das Potential der elektrochemischen Durchflußzelle wurde bei -300 mV vs. SCE konstant gehalten, wobei ein maximaler Stromfluß von 37 mA erreicht wurde. Die Temperatur betrug 37±0,5°C. Der pH-Wert wurde mit 4 N Natronlauge bei 8,5 konstant gehalten.

Nach 70 h waren 96,6 % (16,9 mMol) des D-Lactats zu Pyruvat oxidiert. D-Lactat konnte nicht mehr nachgewiesen werden (≤ 0,01 mMol). Propionsäure war dabei nicht entstanden.

### Beispiel 16

### Isolierung des Pyruvats aus den Reaktionslösungen

Die Reaktionslösungen wurde mit Perchlorsäure auf pH ≤ 2,0 angesäuert und durch Zentrifugation vom Protein befreit. Der Überstand wurde danach 1 h aufgekocht, abgekühlt, mit Natriumchlorid versetzt (Aussalzen) und 6 bis 8 h mit Ether in einem Perforator extrahiert. Der Extrakt wurde im Vakuum eingeengt und in ca. 100 ml kaltem Wasser (≤ 4°C) gelöst (bei ca. 0,15 Mol Brenztraubensäure). Die Lösung wurde mit Natronlauge auf pH 6,0 eingestellt und langsam mit Ethanol versetzt. Das ausgefallene Natrium-Pyruvat wurde danach abfiltriert und 24 h gefriergetrocknet. Die Ausbeute beträgt ca. 95 bis 97 % an Natrium-Pyruvat [Price and Levintow, Biochem. Prepar. 2, 22 (1952)].

### Beispiel 17 (illustrativ)

### Umsetzung einer racemischen 2-Hydroxycarbonsäure mit Proteus mirabilis und Dimethylsulfoxid als Elektronenakzeptor

In einem Rührgefäß wurden 12,5 mMol (D,L)-Hydroxy-4-phenyl-3E-butenoat, 0,5 mMol Anthrachinon-2,6-disulfonsäure, 6,5 mMol Dimethylsulfoxid in 50 ml entionisiertem Wasser gelöst und 170 mg Proteus mirabilis-Zellen (Trockenmasse) zugegeben. Die Temperatur betrug 40±0,5°C. Der pH-Wert wurde bei 8,5 konstant gehalten. Nach 6 h waren 81 % (5,1 mMol) und nach 23,5 h > 99 % (6,2 mMol) des (D)-2-Hydroxy-4-phenyl-3E-butenoats zu 2-Oxo-4-phenyl-3E-butenoat oxidiert. Das (L)-2-Hydroxy-4-phenyl-3E-butenoat dagegen war noch vollständig vorhanden.

### Beispiel 18 (illustrativ)

### Bildung von 2-Oxo-(D)-gluconat aus (D)-Gluconat mit Proteus mirabilis und Dimethylsulfoxid als Elektronenakzeptor

In einem Rührgefäß wurden 10,0 mMol (D)-Gluconat, 0,05 mMol Anthrachinon-2,6-disulfonsaure, 0,25 mMol EDTA, 15,0 mMol Dimethylsulfoxid in 50 ml entionisiertem Wasser gelöst und 800 mg Proteus mirabilis-Zellen (Trockenmasse) zugegeben.

Die Temperatur betrug 40±0,5°C. Der pH-Wert wurde bei 9,3±0,1 konstant gehalten. Nach < 20 h waren 99 % (9,9 mMol) des (D)-Gluconats zu 2-Oxo-(D)-gluconat oxidiert.

## Patentansprüche

1. Verfahren zur Herstellung von Brenztraubensäure aus (D)-Milchsäure unter Verwendung eines Biokatalysators, dadurch gekennzeichnet, daß man die bei der Oxidation von (D)-Milchsäure entstehenden Elektronen auf Anthrachinonsulfonsäure als Mediator überträgt und anschließend den Mediator durch Reaktion mit einem Elektronenakzeptor reoxidiert, wobei die Eduktkonzentration zwischen 0,01 bis 0,7 M liegt.

2. Verfahren zur Herstellung von Mischungen von Brenztraubensäure und (L)-Milchsäure aus (D,L)-Milchsäure unter Verwendung eines Biokatalysators, dadurch gekennzeichnet, daß man die bei der Oxidation von (D)-Milchsäure entstehenden Elektronen auf Anthrachinonsulfonsäure als Mediator überträgt und anschließend den Mediator durch Reaktion mit einem Elektronenakzeptor reoxidiert, wobei die Eduktkonzentration zwischen 0,01 bis 0,7 M liegt.

## Revendications

1. Procédé pour préparer l'acide pyruvique à partir de l'acide (D)-lactique avec utilisation d'un biocatalyseur caractérisé par le fait que l'on transfère les électrons apparus à l'oxydation de l'acide (D)-lactique à un acide anthraquinonesulfonique qui sert de médiateur puis on réoxyde le médiateur par réaction avec un accepteur d'électrons, à une concentration en composant de départ allant de 0,01 à 0,7 M.

2. Procédé pour la préparation de mélanges d'acide pyruvique et d'acide (L)-lactique à partir de l'acide (D,L)-lactique avec utilisation d'un biocatalyseur, caractérisé par le fait que l'on transfère les électrons apparus à l'oxydation de l'acide (D)-lactique à un acide anthraquinonesulfonique qui sert de médiateur puis on réoxyde le médiateur par réaction avec un accepteur d'électrons, à une concentration en composant de départ allant de 0,01 à 0,7 M.

## Claims

1. A process for preparing pyruvic acid from (D)-lactic acid using a biocatalyst, which comprises transferring the electrons which are produced in the oxidation of (D)-lactic acid to anthraquinonesulfonic acid as mediator, and subsequently reoxidizing the mediator by reaction with an electron acceptor, the precursor concentration being from 0.01 to 0.7 M.

2. A process for preparing mixtures of pyruvic acid and (L)-lactic acid from (D,L)-lactic acid using a biocatalyst, which comprises transferring the electrons which are produced in the oxidation of (D)-lactic acids to anthraquinonesulfonic acid as mediator, and subsequently reoxidizing the mediator by reaction with an electron acceptor, the precursor concentration being from 0.01 to 0.7 M.
